# EUROPEAN PATENT APPLICATION

(11) **EP 2 123 653 A1**
(43) Date of publication of application: **25.11.2009**
(21) Application number: 07860200.0
(22) Date of filing: 26.12.2007
(51) Int. Cl.: C07D 473/30, C07H 19/167, C07H 19/173

(54) **PROCESS FOR PRODUCTION OF CRYSTAL OF PURINE NUCLEOSIDE COMPOUND**

(30) Priority: 19.01.2007 JP 2007010494
(71) Applicant: Ajinomoto Co., Inc., Tokyo 104-8315 (JP)
(72) Inventor: NAITO, Masaki, Kawasaki-shi Kanagawa 210-8681 (JP); KIMURA, Yoshitomo, Kawasaki-shi Kanagawa 210-8681 (JP); UEDA, Hiroya, Kawasaki-shi Kanagawa 210-8681 (JP); HARADA, Minoru, Kawasaki-shi Kanagawa 210-8681 (JP)
(74) Representative: Nicholls, Kathryn Margaret
(86) International application number: PCT/JP2007/074971
(87) International publication number: WO 2008/087839

(57) **Abstract**

Disclosed is a process for producing a crystal of a purine nucleoside compound, particularly a crystal of 2',3'-dideoxyinosine, which is not decomposed during storage and has excellent storage stability. Specifically disclosed is a process for producing a crystal of a purine nucleoside which has a concentration of phosphate attached to the crystal of 25 ppm or more and has excellent storage stability. The process comprises the steps of: (1) preparing an aqueous solution containing a phosphate ion (PO₄³⁻) and a purine nucleoside compound; and (2) crystallizing out the purine nucleoside compound from the aqueous solution.

## Description

### Technical Field of the Invention

The present invention relates to a method for producing crystals of a purine nucleoside compound which have excellent storage stability, and particularly it relates to methods for producing crystals of 2',3'-dideoxyinosine (DDI) which are useful as a medical drug such as an anti-AIDS drug.

### Background of the Invention

A 2',3'-dideoxypurine nucleoside compound has an antiviral action which is usable for an anti-AIDS drug, for example. Therefore, it can be used as a medical drug (for example, see Patent Literature 1 and Non-patent Literature 1). Various methods for producing a 2',3'-dideoxypurine nucleoside compound have been reported until now (for example, see Patent Literatures 2 to 7).

However, crystals of 2',3'-dideoxyinosine are decomposed during storage to produce hypoxanthine, and therefore it has been found that there is a problem in the long-term storage stability thereof.
Patent Literature 1: JP-A 61-280500
Patent Literature 2: JP-A 01-224390
Patent Literature 3: JP-A02-117689
Patent Literature 4: JP-A 02-291291
Patent Literature 5: JP-A 03-227997
Patent Literature 6: JP-A 06-041129
Patent Literature 7: JP-A 06-041130
Non-patent Literature 1: J. Med. Chem., 30, 440 (1987)

### Disclosure of the Invention

The object of the present invention is to provide methods for producing crystals of a purine nucleoside compound, and particularly crystals of 2',3'-dideoxyinosine which have excellent storage stability by preventing the crystals from decomposing during storage.

The inventors thoroughly studied methods for producing crystals of a purine nucleoside compound to solve the above problem, and found that excellent storage stability can be obtained by regulating the concentration of phosphate attached to crystals of a purine nucleoside compound within a specific range. The present invention has been completed based on this finding.

Namely, the present invention provides a method for producing crystals of a purine nucleoside compound which have the concentration of phosphate attached to the crystals of 25ppm or more and have excellent storage stability; said method comprising the steps of: (1) preparing an aqueous solution comprising a phosphate ion (PO₄³⁻) and a purine nucleoside compound; and (2) crystallizing out the purine nucleoside compound from the aqueous solution.

### Best Mode for Carrying out the Invention

The method for producing crystals of a purine nucleoside compound of the present invention comprise the step of preparing an aqueous solution comprising a phosphate ion (PO₄³⁻) and a purine nucleoside compound.

Examples of the purine nucleoside compound crystallized by the production method of the present invention include inosine, guanosine, adenosine, and 2',3'-dideoxy form thereof. Among them, 2',3'-dideoxypurine nucleoside compounds such as 2',3'-dideoxy forms of inosine, guanosine and adenosine are useful in the production method of the present invention. Particularly, 2',3'-dideoxyinosine is useful in the production method of the present invention.

Crystals of a purine nucleoside compound obtained by the production method of the present invention preferably have the concentration of phosphate attached to the crystals of 25ppm or more, for example, 25ppm to 2000ppm. It is possible to improve the storage stability of crystals of a purine nucleoside compound by regulating the concentration of the phosphate within such a range. The concentration of the phosphate is more preferably 50ppm or more, for example, 50ppm to 2000ppm.

The concentration of a phosphate ion in an aqueous solution comprising a purine nucleoside compound is preferably 3g/L or more, for example, 3g to 20g/L. It becomes easier to regulate the concentration of the phosphate attached to the finally obtained crystals of a purine nucleoside compound within an appropriate range by regulating the concentration of a phosphate ion within such a range. The concentration of a phosphate ion is more preferably 5g/L or more, for example, 5g to 20g/L.

Next, the method for producing the crystals of the purine nucleoside compound of the present invention comprise the step of crystallizing out the purine nucleoside compound from the aqueous solution.

The crystallization method of the purine nucleoside compound are not particularly limited, and the purine nucleoside compound can be crystallized out by using a generally known crystallization device and method.

Further, the method for producing the crystals of the purine nucleoside compound of the present invention may comprise the step of washing the obtained crystals of a purine nucleoside compound with water or an aqueous solution comprising a phosphate ion.

Washing of the crystals of the purine nucleoside compound with water or an aqueous solution comprising a phosphate ion is conducted so that the concentration of the phosphate attached to the crystals of the purine nucleoside compound is 25ppm or more, for example, 25ppm to 2000ppm, and more preferably 50ppm or more, for example, 50ppm to 2000ppm. The concentration of the phosphate ion of the aqueous solution comprising the phosphate ion for washing the crystals of a purine nucleoside compound is preferably 3g/L or more, for example, 3g/L to 20g/L. It becomes easier to regulate the concentration of the phosphate attached to the finally obtained crystals of the purine nucleoside compound within an appropriate range by regulating the concentration of a phosphate ion within such a range. The concentration of the phosphate ion is more preferably 5g/L or more, for example, 5g/L to 20g/L.

The concentration of the phosphate attached to the crystals of the purine nucleoside compound can be measured by the method comprising the steps of dissolving the crystals with water, and measuring the concentration thereof with a column chromatography described in this specification. The weight ratio of the measured phosphate to the crystals of the purine nucleoside compound is regarded as the concentration of the phosphate attached to the crystals.

### Examples

### (Examples 1 to 4 and Comparative Examples 1 to 4)

2',3'-dideoxyinosine was dissolved in an aqueous solution comprising 10g/L of a phosphate ion (PO₄³⁻) to obtain an aqueous solution comprising 100g/L of 2',3'-dideoxyinosine. Then, crystals of 2',3'-dideoxyinosine were isolated by concentration crystallization, and the thus obtained crystals were washed with water. The following Table 1 shows the concentration of the phosphate attached to the surface of the obtained crystals of 2',3'-dideoxyinosine.

In order to examine the storage stability, the obtained crystals were stored at 60°C, at RH75% for 7 days. The following Table 1 also shows an increase of hypoxanthine in the crystals of 2',3'-dideoxyinosine after storage. For comparison, Table 1 also shows data on crystals of 2',3'-dideoxyinosine which were produced by the same method as the above one except that 2',3'-dideoxyinosine was dissolved in an aqueous solution not comprising a phosphate ion (PO₄³⁻).

**Table 1**

| | Phosphate radical conc. on the surface of crystals (ppm/DDI) | Increase of hypoxanthine after storage (%/DDI) |
|---|---|---|
| Exam. 1 | 480 | 0.45 |
| Exam. 2 | 90 | 0.48 |
| Exam. 3 | 150 | 0.20 |
| Exam. 4 | 140 | 0.54 |
| Cmp.Exam.1 | 0 | 2.24 |
| Cmp.Exam.2 | 0 | 1.91 |
| Cmp.Exam.3 | 0 | 2.21 |
| Cmp.Exam.4 | 0 | 2.20 |

From the above results, it is clarified that the increase of hypoxanthine of each of Examples 1 to 4 is less than that of each of Comparative Examples 1 to 4 which have the concentration of the phosphate on the surface of the crystals of 0ppm.

The concentration of the phosphate on the surface of the crystals and the amount of hypoxanthine were analyzed in the conditions of the following Table 2

**Table 2**

| | Phosphate radical conc. | Hypoxanthine amount |
|---|---|---|
| Device | DIONEX ICS-1500 | HPLC |
| Column | Ionpac AS12A 4×200mm | Shiseido CAPCELLPAK C18 4.6 × 250mm |
| Column temp. | 35°C | 40°C |
| Suppressor | ASRS-ULTRA II-4mm | - |
| Suppressor current v. | 60mA | - |
| Analysis time | 15min | 15min |
| Eluent/mobile phase | 2. 7mM Na₂CO₃, 0. 3mM NaHCO₃ | Distilled water: acetonitrile (92.5 : 7.5) |
| Eluent/mobile ph. flow | 1.5ml/min | 1.2ml/min |
| Detector | Conductance meter | Absorptiometer (254nm) |

### (Comparative Example 5)

The crystals of 2',3'-dideoxyinosine of Example 3 were further suspended in water to remove the phosphate attached to the surface thereof. Using the obtained crystals of 2',3'-dideoxyinosine, the storage stability thereof was examined by the same method as that of Example 1. The following Table 3 shows results thereof together with those of Example 3.

**Table 3**

| | Phosphate radical conc. on the surface of crystals (ppm/DDI) | Increase of hypoxanthine after storage (%/DDI) |
|---|---|---|
| Exam. 3 | 150 | 0.20 |
| Cmp.Exam.5 | 0 | 4.32 |

From the above results it is clarified that though crystals of 2',3'-dideoxyinosine were crystallized out from an aqueous solution comprising a phosphate ion, when the phosphate attached to the surface of the crystals were removed by washing them with water, the hypoxanthine amount thereof increases.

### (Examples 5 to 13 and Comparative Examples 6 to10)

2',3'-dideoxyinosine was dissolved in an aqueous solution comprising 10g/L of a phosphate ion (PO₄³⁻) to obtain an aqueous solution comprising 100g/L of 2',3'-dideoxyinosine. Then, crystals of 2',3'-dideoxyinosine were isolated by concentration crystallization, and the thus obtained crystals were washed with water. At that time, the degree of washing was changed per each sample, and the samples having the concentration of the phosphate attached to the crystals of 2',3'-dideoxyinosine as shown in the following Table 4 were obtained. Using the obtained crystals of 2',3'-dideoxyinosine, the storage stability was examined by the same method as that of Example 1. The following Table 4 shows results thereof.

**Table 4**

| | Phosphate radical conc. on the surface of crystals (ppm/DDI) | Increase of hypoxanthine after storage (%/DDI) |
|---|---|---|
| Cmp. Exam. 6 | 0 | 2.47 |
| Cmp. Exam. 7 | 0 | 2.24 |
| Cmp. Exam. 8 | 0 | 2.20 |
| Cmp. Exam. 9 | 6 | 2.21 |
| Cmp.Exam.10 | 8 | 1.91 |
| Example 5 | 26 | 1.38 |
| Example 6 | 29 | 1.03 |
| Example 7 | 41 | 1.19 |
| Example 8 | 45 | 0.90 |
| Example 9 | 52 | 0.70 |
| Example 10 | 76 | 0.97 |
| Example 11 | 118 | 0.90 |
| Example 12 | 138 | 1.26 |
| Example 13 | 173 | 1.04 |

From the above results, it is clarified that the increase of hypoxanthine of each of Comparative Examples 6 to 10 having the concentration of the phosphate on the surface of the crystals of less than 25ppm is more than that of each of Examples 5 to 13 having the concentration of the phosphate on the surface of the crystals of 25ppm or more.

## Claims

1. A method for producing crystals of a purine nucleoside compound which have the concentration of phosphate attached to the crystals of 25ppm or more and have excellent storage stability;
said method comprising the steps of: (1) preparing an aqueous solution comprising a phosphate ion (PO₄³⁻) and a purine nucleoside compound; and (2) crystallizing out the purine nucleoside compound from the aqueous solution.

2. The method for producing the crystals of the purine nucleoside compound according to claim 1, which further comprises the step of washing the crystallized purine nucleoside compound with water or an aqueous solution comprising a phosphate ion.

3. The method for producing the crystals of the purine nucleoside compound according to any one of claims 1 and 2, wherein the purine nucleoside compound is a 2',3'-dideoxypurine nucleoside compound.

4. The method for producing the crystals of the purine nucleoside compound according to claim 3, wherein the 2',3'-dideoxypurine nucleoside compound is 2',3'-dideoxyinosine.

5. The method for producing the crystals of the purine nucleoside compound according to any one of claims 1 to 4, wherein the concentration of the phosphate attached to the surface of the purine nucleoside compound is 50ppm or more.
